(19) Europäisches Patentamt
European Patent Office
Office européen des brevets

(11) **EP 2 470 222 B1**

(12) **EUROPÄISCHE PATENTSCHRIFT**

(45) Veröffentlichungstag und Bekanntmachung des Hinweises auf die Patenterteilung:
**14.10.2015 Patentblatt 2015/42**

(21) Anmeldenummer: **10742503.5**

(22) Anmeldetag: **13.08.2010**

(51) Int Cl.:
*A61L 15/60* (2006.01)     *A61L 15/22* (2006.01)
*C08F 2/10* (2006.01)     *C08F 2/34* (2006.01)
*C08F 220/06* (2006.01)     *C08F 2/18* (2006.01)
*C08F 2/22* (2006.01)     *C08F 222/10* (2006.01)

(86) Internationale Anmeldenummer:
**PCT/EP2010/061802**

(87) Internationale Veröffentlichungsnummer:
**WO 2011/023572 (03.03.2011 Gazette 2011/09)**

(54) **VERFAHREN ZUR HERSTELLUNG WASSERABSORBIERENDER POLYMERPARTIKEL MIT VERBESSERTER BLUTABSORPTION DURCH POLYMERISATION VON TROPFEN EINER MONOMERLÖSUNG**

METHOD FOR PRODUCING WATER-ABSORBING POLYMER PARTICLES HAVING IMPROVED BLOOD ABSORPTION BY POLYMERISING DROPLETS OF A MONOMER SOLUTION

PROCÉDÉ DE PRODUCTION DE PARTICULES POLYMÉRIQUES ABSORBANT L'EAU À ABSORPTION DU SANG AMÉLIORÉE PAR POLYMÉRISATION DE GOUTTES D'UNE SOLUTION DE MONOMÈRE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **25.08.2009 US 236544 P**

(43) Veröffentlichungstag der Anmeldung:
**04.07.2012 Patentblatt 2012/27**

(73) Patentinhaber: **BASF SE**
**67056 Ludwigshafen (DE)**

(72) Erfinder:
• **LOPEZ VILLANUEVA, Francisco Javier**
**67105 Schifferstadt (DE)**
• **LINSENBÜHLER, Markus**
**69121 Heidelberg (DE)**
• **DOBRAWA, Rainer**
**70469 Stuttgart (DE)**
• **SIEGEL, Bernd**
**67166 Otterstadt (DE)**
• **WEISMANTEL, Matthias**
**63637 Jossgrund (DE)**

(56) Entgegenhaltungen:
**WO-A1-2008/009598     WO-A1-2008/009599**

**EP 2 470 222 B1**

**Beschreibung**

Die vorliegende Erfindung betrifft ein Verfahren zur Herstellung wasserabsorbierender Polymerpartikel mit verbesserter Blutabsorption durch Polymerisation von Tropfen einer Monomerlösung in einer umgebenden Gasphase, wobei die Monomerlösung ein Tensid enthält.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet. Wasserabsorbierende Polymere werden auch als "superabsorbent polymers" bzw. "Superabsorber" bezeichnet.

Durch Sprühpolymerisation können die Verfahrensschritte Polymerisation und Trocknung zusammengefasst werden. Zusätzlich kann die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP 0 348 180 A1, EP 0 816 383 A1, WO 96/40427 A1, US 4,020,256, US 2002/0193546 und DE 35 19 013 A1 beschrieben.

WO 2005/042042 A1 lehrt, dass wasserabsorbierender Polymerpartikel zur Verbesserung der Blutabsorption mit Tensiden und Alkoholen beschichtet werden.

Aufgabe der vorliegenden Erfindung war die Bereitstellung eines verbesserten Verfahrens zur Herstellung wasserabsorbierender Polymerpartikel mit verbesserter Blutabsorption.

Gelöst wurde die Aufgabe durch ein Verfahren zur Herstellung wasserabsorbierender Polymerpartikel durch Polymerisation von Tropfen einer Monomerlösung, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator
d) optional ein oder mehrere mit den unter a) genannten Monomeren copolymerisierbare ethylenisch ungesättigte Monomere,
e) optional ein oder mehrere wasserlösliche Polymere und
f) Wasser,

in einer umgebenden Gasphase, wobei die Monomerlösung mindestens ein Tensid enthält.

Das mindestens eine Tensid kann ein anionisches, kationisches und/oder nichtionisches Tensid sein. Nichtionische Tenside sind bevorzugt, insbesondere nichtionische Tenside mit einem HLB-Wert von 2 bis 18. Der HLB-Wert ist ein Maß für die Wasser- bzw. Öl-Löslichkeit von vorwiegend nichtionischen Tensiden und kann nach üblichen Methoden bestimmt werden.

Ein Tensid besteht aus mindestens einer unpolaren und mindestens einer polaren Gruppe. Bevorzugte Tenside weisen große unpolare und/oder polare Gruppen auf. Große Gruppen sind Gruppen mit einem Molgewicht von mindestens 130 g/mol, vorzugsweise mindestens 250 g/mol, besonderes bevorzugt mindestens 500 g/mol.

Geeignete Tenside sind beispielsweise Sorbitanester, wie Sorbitanmonostearat, Sorbitanmonooleat, Sorbitanpalmitat und Sorbitallaurat sowie Glycerinester, deren Säurekomponente sich von C14- bis C20-Carbonsäuren ableitet.

Bevorzugte Tenside sind alkoxilierte, vorzugsweise ethoxilierte, Alkohole, wobei die Alkohole ggf. verzweigt und/oder ungesättigt sein können, sowie alkoxilierte, vorzugsweise ethoxilierte, Sorbitanmonoester, wie Sorbitanmonostearat und Sorbitanmonooleat.

Ganz besonders bevorzugte Tenside sind ethoxilierte C8-C20-Alkohole.

Das mindestens eine Tensid hat vorzugsweise eine Viskosität von über 20 mPas, besonders bevorzugt von über 25 mPas, ganz besonders bevorzugt von über 30 mPas (gemessen bei 23°C gemäß EN12092).

Die im erfindungsgemäßen Verfahren einzusetzende Menge an Tensid beträgt vorzugsweise von 0,001 bis 5 Gew.-%, besonders bevorzugt von 0,01 bis 2 Gew.-%, ganz besonders bevorzugt von 0,1 bis 1 Gew.-%, jeweils bezogen auf das Monomer a).

Durch den Zusatz des Tensids zur Monomerlösung wird die Blutabsorption im Vergleich zur bislang üblichen nachträglichen Beschichtung erheblich gesteigert und die Aufnahmezeit für Blut deutlich verringert.

Im Folgenden wird die Herstellung der, üblicherweise wasserunlöslichen, wasserabsorbierenden Polymerpartikel näher erläutert.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, Maleinsäure und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu mindestens 25 mol-%, bevorzugt zu 50 bis 80 mol-%, besonders bevorzugt 60 bis 75 mol-%, ganz besonders bevorzugt 65 bis 72 mol%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich. Optional können der Monomerlösung oder ihren Ausgangsstoffen ein oder mehrere Chelatbildner zur Maskierung von Metallionen, wie beispielsweise Eisen, zwecks Stabilisierung zugesetzt werden. Geeignete Chelatbildner sind beispielsweise Alkalicitrate, Zitronensäure, Alkalitartrate, Pentanatriumtriphosphat, Ethylendiamintetraazetat, Nitrilotriessigsäure, sowie alle unter dem Namen Trilon® bekannten Chelatbildner, wie beispielsweise Trilon® C (Pentanatriumdiethylentriaminpentaazetat), Trilon® D (Trinatrium-(hydroxyethyl)-ethylen-diamintriazetat), sowie Tri-Ion® M (Methylglycindiessigsäure).

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden. Die Hydrochinonhalbether können aber auch durch Absorption, beispielsweise an Aktivkohle, aus der Monomerlösung entfernt werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15 bis 30-fach ethoxiliertes Glyzerintriacrylat, 15 bis 30-fach ethoxiliertes Trimethylolpropantriacrylat, 15 bis 20-fach ethoxiliertes Trimethyloletanthantriacrylat, 15 bis 20-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,01 bis 1,5 Gew.-%, besonders bevorzugt 0,05 bis 1 Gew.-%, ganz besonders bevorzugt 0,1 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 $g/cm^2$ (AUL0.3psi) durchläuft ein Maximum.

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die so genannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis-[2-(2-imidazolin-2-yl)propan]-dihydrochlorid und 2,2'-Azobis-[2-(5-methyl-2-imidazolin-2-yl)propan]-dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 2 Gew.-%, bezogen auf die Monomeren a).

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Der Wassergehalt der Monomerlösung beträgt vorzugsweise weniger als 65 Gew.-%, bevorzugt weniger als 62 Gew.-%, besonders bevorzugt weniger als 60 Gew.-%, ganz besonders bevorzugt weniger als 58 Gew.-%.

Die Monomerlösung hat bei 20°C eine Dichte von vorzugsweise 1 bis 1,3 $g/cm^3$, besonders bevorzugt 1,05 bis 1,25 $g/cm^3$, besonders bevorzugt 1,1 bis 1,2 $g/cm^3$.

Die wässrige Monomerlösung wird unter Ausbildung diskreter Tropfen in eine Gasphase dosiert.

Im erfindungsgemäßen Verfahren können eine oder mehrere Sprühdüsen eingesetzt werden. Die einsetzbaren Sprühdüsen unterliegen keiner Beschränkung. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland GmbH, DE).

Erfindungsgemäß bevorzugt sind Vollkegeldüsen. Darunter sind solche mit einem Öffnungswinkel des Sprühkegels von 60 bis 180° bevorzugt. Besonders bevorzugt sind Öffnungswinkel von 90 bis 120°. Der Durchsatz je Sprühdüse beträgt zweckmäßig 0,1 bis 10 $m^3$/h, häufig 0,5 bis 5 $m^3$/h.

Die Reaktion kann auch in Apparaten durchgeführt werden in denen die Monomerlösung in Form monodisperser Tropfen frei fallen kann. Geeignet dazu sind Apparaturen, wie sie beispielsweise in US 5,269,980 beschrieben sind.

Eine Tropfenerzeugung durch laminaren Strahlzerfall, wie in Rev. Sci. Instr. 38 (1966) 502 beschrieben, ist ebenfalls möglich.

Die Tropfen können aber auch mittels pneumatischer Ziehdüsen, Rotation, Zerschneiden eines Strahls oder schnell ansteuerbarer Mikroventildüsen erzeugt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Tropfenerzeugung durch Rotation tritt die Flüssigkeit durch die Öffnungen einer rotierenden Scheibe. Durch die auf die Flüssigkeit wirkende Fliehkraft werden Tropfen definierter Größe abgerissen. Bevorzugte Vorrichtungen zur Rotationsvertropfung werden beispielsweise in DE 43 08 842 A1 beschrieben.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

In einer besonders bevorzugten Ausführungsform der vorliegenden Erfindung wird die Monomerlösung mittels mindestens einer Bohrung unter Ausbildung von Tropfen in den Reaktionsraum dosiert. Die Bohrungen können sich beispielsweise in einer Vertropferplatte befinden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird. In einer bevorzugten Ausführungsform wird die Vertropferplatte nicht angeregt.

Die Anzahl und die Größe der Bohrungen werden gemäß der gewünschten Kapazität und Tropfengröße ausgewählt. Der Tropfendurchmesser beträgt dabei üblicherweise das 1,9-fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Die Reynoldszahl bezogen auf den Durchsatz pro Bohrung und den Bohrungsdurchmesser ist vorzugsweise kleiner als 2.000, bevorzugt kleiner 1.600, besonders bevorzugt kleiner 1.400, ganz besonders bevorzugt kleiner 1.200.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der so genannten Dreiecksteilung, d.h. jeweils drei Bohrungen bilden die Ecken eines gleichseitigen Dreiecks.

Der Abstand der Bohrungen beträgt vorzugsweise 1 bis 50 mm, besonders bevorzugt 2,5 bis 20 mm, ganz besonders bevorzugt 5 bis 10 mm.

Die Temperatur der Monomerlösung beim Durchtritt durch die Bohrung beträgt vorzugsweise 10 bis 60°C, besonders bevorzugt 15 bis 50°C, ganz besonders bevorzugt 20 bis 40°C.

Der Reaktionsraum wird von einem Gas durchströmt. Dabei kann das Trägergas im Gleichstrom oder im Gegenstrom zu den frei fallenden Tropfen der Monomerlösung durch den Reaktionsraum geführt werden, bevorzugt im Gleichstrom, d.h. von oben nach unten. Vorzugsweise wird das Gas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Der Sauerstoffgehalt des Trägergases beträgt vorzugsweise von 0,5 bis 15 Vol.-%, besonders bevorzugt von 1 bis 10 Vol.-%, ganz besonders bevorzugt von 2 bis 7 Vol.%.

Das Trägergas enthält neben Sauerstoff vorzugsweise Stickstoff. Der Stickstoffgehalt des Trägergases beträgt vorzugsweise mindestens 80 Vol.-%, besonders bevorzugt mindesten 90 Vol.-%, ganz besonders bevorzugt mindestens 95 Vol.-%.

Die Gasgeschwindigkeit wird vorzugsweise so eingestellt, dass die Strömung im Reaktionsraum gerichtet ist, beispielsweise liegen keine der allgemeinen Strömungsrichtung entgegen gesetzte Konvektionswirbel vor, und beträgt beispielsweise 0,01 bis 5 m/s, vorzugsweise 0,02 bis 4 m/s, besonders bevorzugt 0,05 bis 3 m/s, ganz besonders bevorzugt 0,1 bis 2 m/s.

Das den Reaktionsraum durchströmende Gas wird zweckmäßigerweise vor Eintritt in den Reaktionsraum auf die Reaktionstemperatur vorgewärmt.

Die Gaseingangstemperatur, d.h. die Temperatur mit der das Gas in den Reaktionsraum eintritt, beträgt vorzugsweise von 160 bis 250°C, besonders bevorzugt von 180 bis 230°C, ganz besonders bevorzugt von 190 bis 220°C.

Vorteilhaft wird die Gaseintrittstemperatur so geregelt, dass die Gasaustrittstemperatur, d.h. die Temperatur mit der das Gas den Reaktionsraum verlässt von 100 bis 180°C, besonders bevorzugt von 110 bis 160°C, ganz besonders bevorzugt von 120 bis 140°C, beträgt.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktionsraum verlassende Gas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktionsraum zurückgeführt werden. Ein Teil des Reaktionsabgases kann ausgeschleust und durch frisches Gas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleit beheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Die wasserabsorbierenden Polymerpartikel können zur weiteren Verbesserung der Eigenschaften zusätzlich beschichtet oder nachbefeuchtet werden. Geeignete Beschichtungen zur Staubbindung sind beispielsweise Polyole, hyperverzweigte hydrophile Polymere, wie beispielsweise Polyglyzerin, sowie hydrophile Dendrimere. Geeignete Beschichtungen gegen die unerwünschte Verbackungsneigung der Polymerpartikel sind beispielsweise pyrogene Kieselsäure, wie beispielsweise Aerosil® 200, und Tenside, wie beispielsweise Span® 20 (Sorbitanmonolaurat), Rewoderm® S1333 (Dinatriumrizinolsäuremonoethanolamidosulfosuccinat), sowie die in WO 2007/074108 A1 offenbarten Tenside. Besonders geeignet sind N-haltige Tenside, kationische Tenside sowie nicht-ionische Tenside. Geeignete Beschichtungen zur Verbesserung der Farbstabilität (Vergilbungsstabilität) sind beispielsweise Reduktionsmittel, wie Natriumhypophosphit, Natriumsulfit, Natriumhydogensulfit, Brüggolite® FF6 und Brüggolite® FF7 (Brüggemann Chemicals; Heilbronn; DE).

Ein weiterer Gegenstand der vorliegenden Erfindung sind gemäß dem erfindungsgemäßen Verfahren erhältliche wasserabsorbierende Polymerpartikel.

Die erfindungsgemäßen wasserabsorbierenden Polymerpartikel haben vorzugsweise die Form von, teilweise eingedrückten, Hohlkugeln und sind annähernd rund, d.h. die Polymerpartikel weisen eine mittlere Sphärizität (mSPHT) von mindestens 0,84, vorzugsweise mindestens 0,86, besonders bevorzugt mindestens 0,88, ganz besonders bevorzugt mindestens 0,9, auf. Die Sphärizität (SPHT) ist definiert als

$$SPHT = \frac{4\pi A}{U^2},$$

wobei A die Querschnittsfläche und U der Querschnittsumfang der Polymerpartikel ist. Die mittlere Sphärizität (mSPHT) ist die volumengemittelte Sphärizität.

Die mittlere Sphärizität (mSPHT) kann beispielsweise mit dem Bildanalysesystem Camsizer® (Retsch Technolgy GmbH; Haan; DE) bestimmt werden.

Polymerpartikel mit relativ niedriger mittlerer Sphärizität (mSPHT) werden durch umgekehrte Suspensionspolymerisation erhalten, wenn die Polymerpartikel während oder nach der Polymerisation agglomeriert werden.

Die durch übliche Lösungspolymerisation (Gelpolymerisation) hergestellten wasserabsorbierenden Polymerpartikel werden nach der Trocknung gemahlen und klassiert wobei unregelmäßige Polymerpartikel erhalten werden. Die mittlere Sphärizität (mSPHT) dieser Polymerpartikel beträgt zwischen ca. 0,72 und ca. 0,78.

Die erfindungsgemäßen wasserabsorbierenden Polymerpartikel weisen einen Gehalt an hydrophobem Lösungsmittel von typischerweise weniger als 0,005 Gew.-%, vorzugsweise weniger als 0,002 Gew.-%, besonders bevorzugt weniger als 0,001 Gew.%, ganz besonders bevorzugt weniger als 0,0005 Gew.-%, auf. Der Gehalt an hydrophobem Lösungsmittel kann gaschromatographisch bestimmt werden, beispielsweise mittels der Head-Space-Technik.

Polymerpartikel, die durch umgekehrte Suspensionspolymerisation erhalten wurden, enthalten üblicherweise noch ca. 0,01 Gew.-% des als Reaktionsmediums verwendeten hydrophoben Lösungsmittels.

Die erfindungsgemäßen wasserabsorbierenden Polymerpartikel weisen eine Blutabsorption von vorzugsweise mindestens 15 g/g, besonders bevorzugt mindestens 18 g/g, ganz besonders bevorzugt mindestens 20 g/g, auf. Die Blutabsorption beträgt üblicherweise weniger als 40 g/g.

Die erfindungsgemäßen wasserabsorbierenden Polymerpartikel weisen eine Aufnahmezeit für Blut von vorzugsweise weniger als 7 Sekunden, besonders bevorzugt weniger als 6 Sekunden, ganz besonders bevorzugt weniger als 5 Sekunden, auf.

Der mittlere Durchmesser der erfindungsgemäßen wasserabsorbierenden Polymerpartikel beträgt vorzugsweise 300 bis 450 $\mu$m, besonders bevorzugt von 320 bis 420 $\mu$m, ganz besonders von 340 bis 400 $\mu$m.

Die erfindungsgemäßen wasserabsorbierenden Polymerpartikel weisen einen Feuchtegehalt von vorzugsweise 5 bis 20 Gew.-%, besonders bevorzugt von 7 bis 18 Gew.%, ganz besonders bevorzugt von 10 bis 16 Gew.-%, auf.

Weitere Gegenstände der vorliegenden Erfindung sind Hygieneartikel, welche die erfindungsgemäßen wasserabsorbierenden Polymerpartikel enthalten.

Unter Hygieneartikel sind dabei insbesondere Damenbinden zu verstehen.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Blutabsorption

Die Blutabsorption wird gemäß der von der EDANA empfohlenen Testmethode Nr. WSP 241.2-05 "Centrifuge Retention Capacity" bestimmt, wobei statt einer 0,9 gew.%igen wässrigen Natriumchloridlösung ein gemäß US 6,147,424 (Spalte 17, Zeile 33 bis Spalte 18, Zeile 45) modifiziertes Schafblut verwendet wird.

Tropfentest

Mit dem Tropfentest wird die Aufnahmezeit für Blut bestimmt. Zur Messung wir eine ca. 1 mm hohe Schicht wasserabsorbierender Polymerpartikel vorgelegt. Mittels einer Eppendorf-Pipette werden 0,1 ml gemäß US 6,147,424 modifizierten Schafbluts aufgetropft und die Zeit bis zum Verschwinden des Tropfens gemessen. Es wird der Mittelwert dreier Messungen berechnet.

Die EDANA-Testmethoden sind beispielsweise erhältlich bei der EDANA, Avenue Eugene Plasky 157, B-1030 Brüssel, Belgien.

Beispiele:

Beispiel 1

18,3 kg wässrige Natriumacrylat-Lösung (37,5 gew.-%ige Lösung in entionisiertem Wasser) und 2,1 kg Acrylsäure wurden mit 13,0 g dreifach ethoxiliertem Glycerintriacrylat (ca. 85gew.-%ig) gemischt. Die Lösung wurde mit Stickstoff inertisiert, so dass der Sauerstoffgehalt auf 6 ppm fiel, und in einem erwärmten Vertropfungsturm vertropft (12 m Höhe, 2 m Breite, Gasgeschwindigkeit 0,1 m/s im Gleichstrom). Die Dosiergeschwindigkeit der Monomerlösung betrug 20,5 kg/h, die Temperatur der Monomerlösung betrug 25°C. Die Vertropferplatte hatte 20 Bohrungen ä 200 $\mu$m. Der Initiator wurde vor dem Vertropfer über statische Mischer in die Monomerlösung dosiert. Als Initiatoren wurden eine 1,8 gew.-%ige wässrige Lösung von 2,2'-Azobis-[2-(2-imidazolin-2-yl)-propan]-dihydrochlorid und eine 3 gew.-%ige wässrige Lösung von Natriumperoxodisulfat verwendet. Die Dosiergeschwindigkeit der Initiatorlösungen betrug 1,031 kg/h bzw. 0,619 kg/h. Mischer und Vertropfer waren direkt miteinander verbunden. Die Heizleistung der Gasvorwärmung wurde so geregelt, dass die Gasausgangstemperatur des Vertropfungsturms 130°C betrug. Die erhaltenen Polymerpartikel wurden auf eine Partikelgröße von 150 bis 850 $\mu$m abgesiebt, um eventuell gebildete Agglomerate abzutrennen.

Die erhaltenen Polymerpartikel wurden analysiert. Die Ergebnisse sind in Tabelle 1 zusammengefasst.

Beispiel 2

Es wurde verfahren wie unter Beispiel 1, zusammen mit der wässrigen Lösung von 2,2'-Azobis-[2-(2-imidazolin-2-yl)-propan]-dihydrochlorid wurden zusätzlich 19 g/h Lutensol® AT80 (BASF SE; Ludwigshafen; DE) in die Monomerlösung dosiert. Lutensol® AT80 ist ein ethoxilierter Alkohol auf Basis eines gesättigten linearen C16-C18-Fettalkohols mit ca. 80 Ethylenoxideinheiten.

Die erhaltenen Polymerpartikel wurden analysiert. Die Ergebnisse sind in Tabelle 1 zusammengefasst.

Beispiel 3

Es wurde verfahren wie unter Beispiel 1, zusammen mit der wässrigen Lösung von 2,2'-Azobis-[2-(2-imidazolin-2-yl)-propan]-dihydrochlorid wurden zusätzlich 37 g/h Lutensol® AT80 in die Monomerlösung dosiert.

Die erhaltenen Polymerpartikel wurden analysiert. Die Ergebnisse sind in Tabelle 1 zusammengefasst.

Beispiel 4

800 g Polymerpartikel aus Beispiel 1 wurden bei Umgebungstemperatur in einen Pflugschar® Mischer Typ M5 mit Heizmantel (Gebr. Lödige Maschinenbau GmbH, Paderborn, DE) gegeben. Bei 200 Upm der Mischerwelle wurde auf die Polymerpartikel innerhalb von 4 Minuten 10,9 g einer 15,0 gew.-%igen wässrigen Lösung von Lutensol® AT80 aufgesprüht. Die Mischerwellengeschwindigkeit wurde dann auf 60 Upm reduziert und es wurde unter diesen Bedingungen noch 5 Minuten nachgemischt. Die beschichteten Polymerpartikel wurden aus dem Mischer ausgetragen und eventuell gebildete Agglomerate wurden mittels eines Siebes mit einer Maschenweite von 850 $\mu$m abgesiebt.

Beispiel 5

Es wurde verfahren wie unter Beispiel 4, wobei 21,9 g der 15,0 gew.-%igen wässrigen Lösung von Lutensol® AT80 aufgesprüht wurden.

Tab. 1: Ergebnisse

| Beispiel | Menge an Tensid bez. auf Acrylsäure | Blutabsorption [g/g] | Tropfentest [s] |
|---|---|---|---|
| 1*) | ohne | 6,8 | 7,0 |
| 2 | 0,25 Gew.-% | 16,0 | 5,3 |
| 3 | 0,5 Gew.-% | 20,7 | 4,0 |
| 4*) | 0,25 Gew.-% | 10,2 | 11,3 |
| 5*) | 0,5 Gew.-% | 10,4 | 11,0 |
| *) Vergleichsbeispiele | | | |

**Patentansprüche**

1. Verfahren zur Herstellung wasserabsorbierender Polymerpartikel durch Polymerisation von Tropfen einer Monomerlösung, enthaltend

   a) mindestens ein ethylenisch ungesättigtes, säuregruppentragendes Monomer, das zumindest teilweise neutralisiert sein kann,
   b) mindestens einen Vernetzer,
   c) mindestens einen Initiator,
   d) optional ein oder mehrere mit den unter a) genannten Monomeren copolymerisierbare ethylenisch ungesättigte Monomere,
   e) optional ein oder mehrere wasserlösliche Polymere und
   f) Wasser,

   in einer umgebenden Gasphase, wobei die Monomerlösung mindestens ein Tensid enthält.

2. Verfahren gemäß Anspruch 1, **dadurch gekennzeichnet, dass** das Monomer a) zu mindestens 50 mol-% Acrylsäure ist.

3. Verfahren gemäß Anspruch 1 oder 2, **dadurch gekennzeichnet, dass** die Säuregruppen des Monomeren a) zu mindestens 25 mol-% neutralisiert sind.

4. Verfahren gemäß einem der Ansprüche 1 bis 3, **dadurch gekennzeichnet**, die Monomerlösung, bezogen auf das Monomer a), von 0,001 bis 5 Gew.% des mindestens eines Tensids enthält.

5. Verfahren gemäß einem der Ansprüche 1 bis 4, **dadurch gekennzeichnet, dass** das mindestens eine Tensid ein ethoxilierter Alkohol ist.

6. Wasserabsorbierende Polymerpartikel, erhältlich gemäß einem Verfahren der Ansprüche 1 bis 5, wobei die Polymerpartikel eine mittlere Sphärizität von mindestens 0,84 aufweisen.

7. Polymerpartikel gemäß Anspruch 6, wobei die Polymerpartikel eine Blutabsorption von mindestens 15 g/g aufweisen.

8. Polymerpartikel gemäß Anspruch 6 oder 7, wobei die Polymerpartikel eine Aufnahmezeit für Blut von weniger als 7 Sekunden aufweisen.

9. Hygieneartikel, enthaltend wasserabsorbierende Polymerpartikel gemäß einem der Ansprüche 6 bis 8.

**Claims**

1. A process for producing water-absorbing polymer particles by polymerising droplets of a monomer solution comprising

a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a),
e) optionally one or more water-soluble polymers, and
f) water,

in a surrounding gas phase, the monomer solution comprising at least one surfactant.

2. The process according to claim 1, wherein the monomer a) is acrylic acid to an extent of at least 50 mol%.

3. The process according to claim 1 or 2, wherein the acid groups of the monomer a) are neutralized to an extent of at least 25 mol%.

4. The process according to any of claims 1 to 3, wherein the monomer solution, based on the monomer a), comprises from 0.001 to 5% by weight of the at least one surfactant.

5. The process according to any of claims 1 to 4, wherein the at least one surfactant is an ethoxylated alcohol.

6. Water-absorbing polymer particles obtainable by a process of claims 1 to 5, said polymer particles having a mean sphericity of at least 0.84.

7. Polymer particles according to claim 6, which have a blood absorbence of at least 15 g/g.

8. Polymer particles according to claim 6 or 7, which have an absorption time for blood of less than 7 seconds.

9. A hygiene article comprising water-absorbing polymer particles according to any one of claims 6 to 8.


**Revendications**

1. Procédé de fabrication de particules polymères absorbant l'eau par polymérisation de gouttes d'une solution de monomères, contenant :

a) au moins un monomère éthyléniquement insaturé, portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères cités en a),
e) éventuellement un ou plusieurs polymères solubles dans l'eau, et
f) de l'eau,

dans une phase gazeuse enrobante, la solution de monomères contenant au moins un tensioactif.

2. Procédé selon la revendication 1, **caractérisé en ce que** le monomère a) est l'acide acrylique à hauteur d'au moins 50 % en moles.

3. Procédé selon la revendication 1 ou 2, **caractérisé en ce que** les groupes acides du monomère a) sont neutralisés à hauteur d'au moins 25 % en moles.

4. Procédé selon l'une quelconque des revendications 1 à 3, **caractérisé en ce que** la solution de monomères contient, par rapport au monomère a), de 0,001 à 5 % en poids dudit au moins un tensioactif.

5. Procédé selon l'une quelconque des revendications 1 à 4, **caractérisé en ce que** ledit au moins un tensioactif est un alcool éthoxylé.

6. Particules polymères absorbant l'eau, pouvant être obtenues par un procédé selon les revendications 1 à 5, dans lesquelles les particules polymères présentent une sphéricité moyenne d'au moins 0,84.

7. Particules polymères selon la revendication 6, dans lesquelles les particules polymères présentent une absorption du sang d'au moins 15 g/g.

8. Particules polymères selon la revendication 6 ou 7, dans lesquelles les particules polymères présentent un temps d'absorption du sang de moins de 7 secondes.

9. Article d'hygiène, contenant des particules polymères absorbant l'eau selon l'une quelconque des revendications 6 à 8.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0348180 A1
- EP 0816383 A1
- WO 9640427 A1
- US 4020256 A
- US 20020193546 A
- DE 3519013 A1
- WO 2005042042 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- US 5269980 A
- DE 4308842 A1
- WO 2007074108 A1
- US 6147424 A

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103
- *Rev. Sci. Instr.,* 1966, 38